# EUROPÄISCHE PATENTSCHRIFT

(11) **EP 0 917 881 B1**
(45) Veröffentlichungstag und Bekanntmachung des Hinweises auf die Patenterteilung: **02.10.2002**
(21) Anmeldenummer: 99103927.2
(22) Anmeldetag: 20.06.1995
(51) Int. Cl.: A61M 1/16

(54) **Vorrichtung zur Herstellung eines flüssigen Hämodialysekonzentrats**
Means for preparing a liquid hemodialysis concentrate
Dispositif pour préparer un concentrat liquide pour l'hémodialyse

(30) Priorität: 24.06.1994 DE 4422100
(43) Veröffentlichungstag der Anmeldung: 26.05.1999
(62) Teilanmeldung aus: 95109497.8
(73) Patentinhaber: Fresenius Medical Care Deutschland GmbH, 61352 Bad Homburg (DE)
(72) Erfinder: Mathieu, Bernd, Dr., 66583 Spiesen (DE)
(74) Vertreter: Luderschmidt, Schüler & Partner GbR

(56) Entgegenhaltungen:
- EP-A- 0 619 135
- US-A- 5 385 564

## Beschreibung

Die Erfindung betrifft eine Vorrichtung zur Herstellung eines flüssigen Hämodialysekonzentrats, die von einem flexiblen Behälter Gebrauch macht, der ausreichend pulverförmiges Konzentratsalz für eine Dialysebehandlung aufweist.

Für die Hämodialyse muß eine Dialysierflüssigkeit bereitgestellt werden, deren Elektrolytzusammensetzung im wesentlichen derjenigen des Bluts des zu behandelnden Patienten entsprechen soll.

Es sind seit langem Verfahren bekannt, mit denen gebrauchsfertige Dialysierflüssigkeiten einstufig aus fertig in Kanistern angelieferten Flüssigkonzentraten hergestellt werden. Gleichermaßen sind Verfahren bekannt, mit denen Dialysierflüssigkeiten in einem Tank bettseitig (ebenso einstufig) aus einem Trockenkonzentrat und Wasser erzeugt werden.

Andererseits kann jedoch aber auch bettseitig zunächst in einer zweistufigen Methode ein Flüssigkonzentrat aus einem Trockenkonzentrat durch Vermischen mit Wasser hergestellt werden, aus dem anschließend durch weiteres Vermischen mit Wasser in einem vorbestimmten Mischungsverhältnis die gewünschte Dialysierflüssigkeit hergestellt wird.

Ein Verfahren der letztgenannten Art ist in der DE 32 12 230 beschrieben, bei dem zunächst ein Trockenkonzentrat in einer Mischkammer vorgelegt wird, die eingangsseitig mit einer Wasserquelle und ausgangsseitig mit einem Dialysierflüssigkeitstank verbunden ist. Hierdurch kann ein individuell angepaßtes Flüssigkonzentrat und daraus eine fertige Dialysierflüssigkeit hergestellt werden. Diese Mischkammer hat im wesentlichen die Aufgabe, nicht nur die leicht löslichen Elektrolytsalze, wie Natriumchlorid, Kaliumchlorid oder Calciumchlorid, sondern auch das in Wasser schwerer lösliche Natriumbicarbonat nur in gelöster Form in die Ausgangsleitung zu überführen. Die Mischung erfolgt, wie bei Batch-Verfahren allgemein üblich, volumetrisch, d.h. eine vorbestimmte Menge Trockenkonzentrat wird mit einem vorbestimmten Volumen Wasser umgesetzt. Bei diesem Mischprinzip wird aus einem Trockenkonzentrat und Wasser ein Flüssigkonzentrat in einen ersten Behälter zumindest zeitweise kontinuierlich und bei einer weiteren Variante aus Flüssigkeit und Wasser eine fertige Dialysierflüssigkeit in dem gleichen oder einem weiteren Behälter zubereitet, wobei dieser Behälter die gesamte Dialysierflüssigkeitsmenge für eine gesamte Behandlung enthält.

In der DE 34 43 911 ist ein Verfahren beschrieben, bei dem ein Flüssigkonzentrat aus Trockenkonzentrat und Wasser in einem Mischbehälter zubereitet wird, der über eine Eingangsleitung und über eine Ausgangsleitung verfügt. Ausgangsseitig kann dann Flüssigkonzentrat einem Mischpunkt zugeführt werden, dem über eine zweite Leitung Wasser in einer vorbestimmten Menge zugeführt wird. Üblicherweise wird in dem Mischbehälter ein Flüssigkonzentrat-Batch für eine gesamte Behandlung erzeugt, so daß im Anschluß daran eine fertige Dialysierflüssigkeit durch kontinuierliche Mischung des Flüssigkonzentrats mit Wasser hergestellt werden kann.

Aus US 4 386 634 ist ein weiteres Verfahren beschrieben, mit dem eine Dialysierflüssigkeit aus Trockenkonzentrat mittels eines zweistufigen Verfahrens hergestellt werden kann, wie es ebenfalls Gegenstand der Erfindung ist. Dabei wird ein Beutel als Mischgefäß für eine batchweise Fertigung von Flüssigkonzentrat benutzt, der während der Behandlung kontinuierlich zu einem Mischpunkt entleert wird, der ebenfalls mit Wasser zur Herstellung einer gebrauchsfertigen Dialysierflüssigkeit beaufschlagt wird. Die batchweise Herstellung von Flüssigkonzentrat hat zwar den Vorteil einer sicheren Mischung des Trockenkonzentrats mit Wasser, andererseits jedoch den Nachteil, daß hierdurch ein relativ großer Raumbedarf für das Konzentrat (8-12 l) notwendig ist, so daß hierdurch stabile Behälter oder aufwendige Lagerungs- und Halterungseinrichtungen notwendig sind. Beide Anordnungen sind dabei mit relativ hohen Kosten für die Desinfektion bzw. für die Bereitstellung verbunden.

Insofern hat man versucht (EP 278 100), Dialysierflüssigkeiten dadurch herzustellen, daß man festes Konzentrat relativ geringen Volumens in einer Kartusche vorgelegt hat, die über einen Wassereinlaß und über einen Flüssigkonzentratauslaß verfügt. Diese Kartusche ist mit einer Monosubstanz, üblicherweise Natriumbicarbonat gefüllt, das mit Wasser von der Kartuscheneingangsseite beaufschlagt wird, so daß sich beim Durchströmen der Flüssigkeit durch die Kartusche hindurch Bicarbonat langsam auflöst und ein Flüssigkonzentrat gebildet wird. Dieses Konzentrat wird durch den Auslaß einem Mischpunkt zugeführt, an dem es mit Wasser und weiteren Flüssigkonzentraten zu einer Dialysierflüssigkeit vorbestimmter Zusammensetzung verdünnt wird.

Dieses kontinuierlich arbeitende Verfahren hat den Vorteil des geringen Raumbedarfs für das Konzentrat und der guten Lagerfähigkeit des Trockenkonzentrats in der Kartusche im Vergleich zu einem in einem Kanister angelieferten Flüssigkonzentrat. Nachteilig an diesem Verfahren und dieser Anordnung sind, daß das Pulver stetig durchflossen werden muß, um die gewünschte Sättigung der Lösung durch Auflösen des Salzes einzustellen. So lösen sich bekanntlich etwa 100 g Natriumbicarbonat bei Raumtemperatur in 1 l Wasser auf. Diese Sättigung ist am Ausgang der Kartusche sicherzustellen, was jedoch durch das häufige Zusammenkleben des Pulvers und die gefürchtete Kanalbildung innerhalb der Kartusche bei ständigem Durchströmen des Wassers häufig nicht sichergestellt werden kann. Insofern ergeben sich aufgrund der unterschiedlichen Zusammensetzung der Flüssigkonzentrate Schwierigkeiten bei der Herstellung der fertigen Dialysierflüssigkeit, was insbesondere bei hohen Mischungsverhältnissen von Wasser zu Flüssigkonzentrat der Fall ist.

Des weiteren sind die Konnektoren von zwei Anschlüssen mit den bekannten Hygiene- und Verwechslungsproblemen behaftet.

Eine weitere Vorrichtung zur kontinuierlichen Herstellung von Flüssigkonzentrat ist in DE 41 39 165 beschrieben, bei der jedoch ebenfalls die vorstehend beschriebenen Kanalbildungs- und Hygiene- sowie Verwechslungsprobleme auftreten können.

Der Erfindung liegt daher die Aufgabe zugrunde, eine Vorrichtung zur Herstellung eines flüssigen Hämodialysekonzentrats zur Verfügung zu stellen, mit der kontinuierlich bettseitig ein gesättigtes flüssiges Konzentrat ohne Kanalbildungs- oder Lagerungsprobleme hergestellt werden kann.

Die erfindungsgemäße Vorrichtung zur Herstellung eines flüssigen Hämodialysekonzentrats macht von einem flexiblen Behälter Gebrauch, der nur ein einziges Anschlußstück aufweist, so daß die Sterilitätsprobleme auf ein Minimum reduziert werden. Des gleichen ist der Inhalt der Verpackungseinheit derartig, daß portionsweise gesättigte Dialysatkonzentrate hergestellt werden können, d.h. während der Behandlung eines Patienten muß die Verpackungseinheit mehrmals mit frischem Wasser aufgefüllt werden, so daß jeweils batchweise Flüssigkonzentrate (Multi-Batch-Verfahren) hergestellt werden.

Die erfindungsgemäße Vorrichtung zur Herstellung eines flüssigen Hämodialysekonzentrats umfaßt eine Wasserquelle mit einem flexiblen Behälter mit einem einzigen Anschlußstück, der ausreichend pulverförmiges Konzentrat fiir eine Dialysebehandlung aufweist, eine Leitungsanordnung, die einerseits die Wasserquelle und andererseits das Anschlußstück sowie eine Einrichtung zur Herstellung einer Dialysierflüssigkeit verbindet, eine Absperreinrichtung, die jeweils eine Wasserquelle, das Anschlußstück und Dialysierflüssigkeitseinrichtung freigibt bzw. sperrt und eine Einrichtung zur Kompensierung des in der Leitungsanordnung befindlichen Totvolumens.

In dieser Anordnung wird im Verhältnis zum Gesamtbedarf an Dialysierflüssigkeit eine relativ kleine Einzelmenge an Konzentrat hergestellt und diese jeweils batchweise zur gebrauchsfertigen Dialysierflüssigkeit verdünnt,

Gemäß einer ersten Ausführungsform weist die Absperreinrichtung Ventile auf, die in der Leitungsanordnung in folgender Weise angeordnet sind. Ein erstes Ventil ist in einem ersten Leitungsstück vorgesehen, das die Wasserquelle mit einem Verknüpfungspunkt verbindet. Von diesem Verknüpfungspunkt geht ein zweites Leitungsstück zum Anschlußstück des flexiblen Behälters ab. In diesem zweiten Leitungsstück ist ein zweites Ventil angeordnet. Des weiteren geht von dem Verknüpfungspunkt ein drittes Leitungsstück ab, das ein drittes Ventil aufweist und an seinem Ende mit der Einrichtung zur Erzeugung der fertigen Dialysierflüssigkeit verbunden ist.

Diese Ventil 1-3 werden gemäß einem vorbestimmten Mischverfahren aktiviert, d.h. geöffnet und anschließend desaktiviert, d.h. geschlossen.

Die Wasserquelle, aus der üblicherweise Umkehrosmose-Wasser (RO-Wasser) bereitgestellt wird, steht üblicherweise unter Druck, so daß zur Zuführung des Wassers im allgemeinen keine Fördereinrichtung in Form einer Pumpe notwendig ist. Andererseits kann jedoch aber auch in der Leitungsanordnung eine Pumpanordnung vorgesehen sein, die frisches Wasser zum flexiblen Behälter fördert und fertiges Flüssigkonzentrat aus dem Behälter wieder abfördert. Ggf. kann die Abförderung auch durch eine in der Einrichtung zur Herstellung der fertigen Dialysierflüssigkeit vorgesehene Pumpe durchgeführt werden.

Gemäß einer ersten Ausführungsform ist das Leitungssystem im Bereich des Verknüpfungspunktes über ein viertes Leitungsstück mit einer volumetrisch arbeitenden Pumpe, üblicherweise einer Proportionierpumpe versehen. Diese Pumpe wirkt dabei sowohl als Zuführungs- als auch als Abführungspumpe. Ihr Innenvolumen ist üblicherweise geringer als das Innenvolumen des flexiblen Behälters, so daß mehrere Pumpvorgänge durchgeführt werden müssen, um den Innenraum mit einer vorbestimmten Wassermenge zu füllen.

Um einen Füllvorgang der Pumpe auszulösen, wird zunächst das erste Ventil geöffnet, während die beiden übrigen Ventile geschlossen sind. Die dann mit Wasser gefüllte Pumpe wird in einem Entleerungsschritt bei geöffentem zweiten Ventil und geschlossenem ersten und dritten Ventil in den flexiblen Behälter entleert. Dieser Vorgang wird so oft durchgeführt, bis eine vorbestimmte Menge Wasser in den vorteilhafterweise als Beutel ausgebildeten flexiblen Behälter überführt worden ist.

Da der flexible Behälter nur über ein einziges Anschlußstück verfügt, weist er auch ein gemeinsames Leitungsstück für das zugeführte Wasser und das abzuführende Flüssigkonzentrat auf. Dieses Totvolumen befindet sich üblicherweise in dem zweiten Leitungsstück zwischen dem Verknüpfungspunkt und dem Anschlußstück des Behälters.

Die dem Behälter jeweils zugeführte Wassermenge reicht üblicherweise nicht aus, um auch nur einen erheblichen Teil des Bicarbonat-Konzentrats im Beutel zu lösen. Üblicherweise werden nur etwa 10-20 % des Konzentrats im Beutel aufgelöst. So wird beispielsweise bei einem Beutel, der ein füllbares Innenvolumen von 1,5 1 aufweist und mit etwa 650 g Natriumbicarbonat und etwa 1 l Wasser gefüllt ist, etwa 100 g Natriumbicarbonat bei Raumtemperatur unter Bildung einer gesättigten Lösung aufgelöst, so daß mindestens weitere fünf Füllund Entleerungsoperationen durchgeführt werden können, ohne daß die Gefahr besteht, die Sättigungskonzentration nicht mehr zu erreichen.

Damit auch tatsächlich das zugeführte Wasser durch Vermischung mit dem pulverförmigen Konzentrat in eine gesättigte Lösung umgewandelt wird, wird vorteilhafterweise die im Beutel befindliche Lösung in Bewegung gehalten. Dies kann beispielsweise dadurch erfolgen, daß der Beutel geschüttelt oder mit Ultraschall behandelt wird, so daß eine innige Durchmischung erfolgt.

Andererseits kann jedoch aber auch die Proportionalpumpe bei geöffnetem zweiten Ventil und permanent geschlossenen Ventilen 1 und 3 mehrfach in die Füll- und Entleerungsphase geschaltet werden, so daß hierdurch eine stoßweise Füllung und Entleerung des Konzentratbeutels erfolgt.

Mit der letzteren Methode, die eine bevorzugte Ausführungsform der Kompensierung des Totvolumens darstellt, wird auch ein weiteres Problem gelöst, nämlich dasjenige des Totvolumens, das sich, wie vorstehend erläutert, im zweiten Leitungsstück sowie den angrenzenden Bereichen einstellt. Diese sind nämlich sowohl von Wasser als auch von Konzentrat durchflossen, so daß jeweils Rückstände, insbesondere diejenigen des Wassers in diesem Totvolumenbereich zu kompensieren sind. Insofern sind vorteilhafterweise die Ventile 1-3 möglichst nahe am Verknüpfungspunkt angeordnet. Wird nun die Pumpe bei geöffnetem zweiten Ventil mehrfach gefüllt und entleert, so hat dies zur Folge, daß das im Totvolumen befindliche Restwasser innig mit bereits gesättigtem Konzentrat in der Pumpe und anschließend im Beutel vermischt wird, so daß im Totvolumenbereich anschließend keine verdünnten Konzentratmengen mehr vorhanden sind.

Andererseits reicht es jedoch aber auch gemäß einer besonders bevorzugten Ausführungsform aus, wenn nach dem letzten Entleerungshub der Proportionierpumpe in der Wasserfüllphase nochmals bei weiter geöffnetem zweitem Ventil ein Füllhub der Pumpe abläuft, so daß das gesamte im Totvolumenbereich befindliche Wasser in die Pumpe und darüber hinaus noch vorteilhafterweise eine geringe Menge an gesättigter Konzentratlösung in die Pumpe abgesaugt wird.

In allen Fällen wird anschließend die Pumpe stillgesetzt, so daß bei geöffnetem zweiten Ventil und dritten Ventil dort gesättigtes Dialysierflüssigkeitskonzentrat bereitgestellt wird, das anschließend in der Einrichtung zur Herstellung einer fertigen Dialysierflüssigkeit aus den übrigen Elektrolytkomponenten weitervermischt wird.

Andererseits läßt sich auch die zu Beginn geförderte Flüssigkeitsmenge an Flüssigkonzentrat einschließlich Totvolumen mittels eines Durchflußsensors hinsichtlich eines seiner Ionenkomponenten (Natriumionen oder Bicarbonationen) quantitativ bestimmen, so daß eine Kompensation durch Regelung der zugeführten Konzentrat-/Wassermengen möglich ist.

Diese Anordnung stellt eine weitere bevorzugte Ausführungsform eine Einrichtung zur Kompensierung des Totvolumens dar. Hier wird fortlaufend die Konzentration in dem zugeführten Wasser/Konzentrat-Gemisch bestimmt und die Einrichtung zur Herstellung der fertigen Dialysierflüssigkeit entsprechend gesteuert. Andererseits kann natürlich auch bei bekanntem Totvolumen eine vorbestimmte Menge Flüssigkeit der Einrichtung zur Herstellung der Dialysierflüssigkeit zugeführt werden, die diese Flüssigkeitsmenge dann nicht als Konzentrat, sondern als zugeführtes Wasser erkennt bzw. akzeptiert. Eine solche Betriebsweise ist bei getaktet arbeitenden volumentrischen Einrichtungen zur Herstellung von Dialysierflüssigkeit einsetzbar, wie es beispielsweise bei dem Dialysegerät 2008 der Anmelderin verwirklicht ist. Hier wird Dialysierflüssigkeit volumetrisch durch Mischen von vorbestimmten Teilen Konzentrat mit Wasser hergestellt. Beispielsweise werden 1 Teil Konzentrat in einen Behälter eingegeben, der anschließend mit 34 Teilen Wasser aufgefüllt wird. Erfindungsgemäß wird die aus dem Totvolumen zunächst zugeführte Flüssigkeitsmenge als Wasser gewertet, d.h. die Pumpenhübe, mit welchen diese Flüssigkeitsmenge zugeführt wird, werden nicht als Konzentrathübe gewertet.

Des weiteren ist es möglich, anstelle der Proportionierpumpe einen Auslaß vorzusehen, durch den dieses Totvolumen in den Abfluß gefördert wird. Hierzu ist vorteilhafterweise eine Ablaßpumpe vorgesehen, die nach der Füllung des Beutels bei geöffnetem zweiten Ventil kurzzeitig betätigt wird. Eine solche Anordnung ist dann vorteilhaft, wenn RO-Wasser unter Druck dem flexiblen Behälter zugeführt werden kann. Diese Pumpe kann natürlich durch ein viertes Ventil ersetzt werden, wenn der Behälter ebenfalls unter Druck entleert werden kann. In einem solchen Fall wird das vierte Ventil so lange geöffnet, bis das Wasser sicher aus Totvolumenbereich entfernt ist und gesättigtes Konzentrat am Abfluß auftaucht.

Grundsätzlich ist dabei festzuhalten, daß das Totvolumen im Verhältnis zu der zugeführten Flüssigkeitsmenge etwa 1-2 % beträgt, so daß die Verluste praktisch vernachlässigbar sind.

Anstelle von Ventilen lassen sich auch Klemmen einsetzen, sofern die Leitungen flexibel sind. Des gleichen können peristaltische Pumpen anstelle dieser Ventile eingesetzt werden, die im desaktivierten Zustand ebenfalls die Leitungen sperren.

Der flexible Behälter, vorzugsweise ein Beutel, der zur Aufnahme des Trockenkonzentrats dient, wird gemäß einer ersten Ausführungsform von unten mit Wasser beaufschlagt, d.h. das Anschlußstück befindet sich bei aufgehängtem Beutel am unteren Ende. Um eine Verschleppung von Trockenkonzentrat nach dem Mischen mit Wasser zu verhindern, werden vorteilhafterweise Abtrenneinrichtungen im Behälter verwendet. So ist beispielsweise die Anschlußöffnung des Behälters mit einer halbdurchlässigen Membran verschlossen, die Wasser und Flüssigkonzentrat, nicht jedoch das Trockenkonzentrat aufgrund seiner Partikelgröße (ca. 0,3 mm und größer) durchläßt. Wird bei dieser Ausführungsform Wasser zugeführt, so findet bereits im Bodenbereich eine innige Durchmischung des Trockenkonzentrats mit Wasser statt, so daß bei dem Durchströmen des Pulvers zur Auffüllung des Behälters sich sofort gesättigtes Konzentrat bildet.

Gemäß einer weiteren Ausführungsform kann der Behälter in umgekehrter Orientierung benutzt werden, d.h. das Anschlußstück befindet sich in der Gebrauchslage oben. In einem solchen Fall wird ein Steigrohr in den Beutel integriert, das bis an die tiefste Stelle des Behälters geführt ist. Dabei ist das Ende des Steigrohrs entweder wiederum mit einer halbdurchlässigen Membran oder aber mit einem Rückschlagventil gesperrt, das nur den Zufluß, nicht jedoch aber den Abfluß von Flüssigkonzentrat zuläßt. Im letzteren Fall befindet sich eine zweite Öffnung benachbart zum Anschlußstück im Steigrohr, das durch ein zweites Rückschlagventil in umgekehrter Richtung zum ersten Rückschlagventil versperrt ist, so daß nur die Abförderung möglich ist.

Die Form und die Anordnung des Konzentrat-Behälters kann weitgehend frei gewählt werden, solange ein ausreichender Kontakt des Wassers mit dem Trockenkonzentrat in der Füllphase gewährleistet ist.

Es zeigen
- Fig. 1: schematisch eine Vorrichtung zur Herstellung eines Flüssigkonzentrats
- Fig. 2: schematisch eine erste Ausführungsform eines Trockenkonzentrat-Beutels,
- Fig. 3: schematisch eine zweite Ausführungsform eines Trockenkonzentrat-Beutels,
- Fig. 4: schematisch eine dritte Ausführungsform eines Trockenkonzentrat-Beutels und
- Fig. 5: schematisch eine vierte Ausführungsform eines Trockenkonzentrat-Beutels.

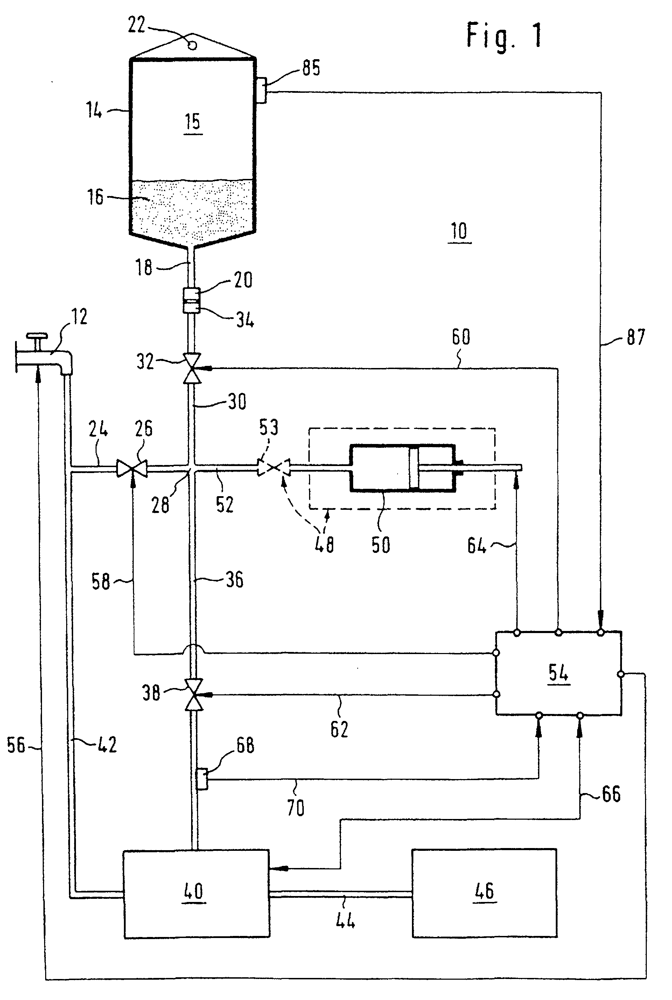

In Fig. 1 ist mit 10 eine Einrichtung zur Herstellung eines Flüssigkonzentrats fiir die Hämodialyse dargestellt. Diese Einrichtung 10 weist eine Wasserquelle 12 auf, die üblicherweise eine stationäre RO-Anlage ist. Aus dieser Wasserquelle kann stetig unter Druck oder druckfrei Wasser zugeführt werden, das im wesentlichen elektrolytfrei ist. Andererseits kann aber auch die Wasserquelle ein

Wasserreservoir sein, das mit so viel Wasser gefüllt ist, daß damit ein Behälter 14 vollständig angefüllt werden kann.

Der Behälter 14 ist flexibel ausgeführt, vorteilhafterweise in Beutelform. Hierzu werden üblicherweise Kunststoffolien eingesetzt, um derartige Beutel herzustellen.

Der Behälter 14 ist mit einem Trockenkonzentrat 16 teilweise gefüllt, das üblicherweise eine Partikelgröße von 0,3 mm und mehr aufweist. Als Trockenkonzentrat können sämtliche bei der Dialyse eingesetzten Elektrolyte in Frage kommen, vorzugsweise das schwer wasserlösliche Natriumbicarbonat. Dieses Trockenkonzentrat liegt in dem Beutel in einer solchen Menge vor, daß mindestens eine Hämodialyse-Behandlung durchgeführt werden kann.

Wird Natriumbicarbonat in dem Behälter 14 vorgelegt, so werden vorteilhafterweise sämtliche anderen Konzentrate (Natriumchlorid, Kaliumchlorid, Calciumchlorid) in einem weiteren, nicht gezeigten Behälter entweder in Trockenform oder in Flüssigkonzentratform vorgelegt.

Üblicherweise enthält der Behälter ca. 500-1 000 g Natriumbicarbonat (je nach herzustellender Gesamtflüssigkeitsmenge). Weiterhin beträgt das Innenvolumen des Behälter 14 das 2-4fache des Ausgangsvolumens des pulverförmigen Natriumbicarbonats bzw. der anderen Trockenkonzentrate.

Der Behälter 14 verfügt über ein einziges rohrförmiges Anschlußstück 18, dessen Ende vorteilhafterweise ein erstes Verbindungsstück 20 aufweist.

Des weiteren ist gemäß der in Fig. 1 gezeigten Ausführungsform auf der gegenüberliegenden Seite des Behälters 14 eine Aufhängeeinrichtung 22, üblicherweise eine Öse vorgesehen, mit der der Behälter 14 an einem nicht gezeigten Stativ aufgehängt werden kann.

Von der Wasserquelle geht ein erstes Leitungsstück 24, das in ein erstes Ventil 26 als Absperrorgan eingeschaltet ist ab und das bis zu einem Verknüpfungspunkt 28 geführt ist. Von dort geht ein zweites Leitungsstück 30, in die ein zweites Ventil 32 eingeschaltet ist und an dessen Ende ein zweites Verbindungsstück 34 angeordnet ist, zum Anschlußstück 18 ab. Das zweite Verbindungsstück wird im Gebrauchsfall mit dem ersten Verbindungsstück 20 konnektiert, so daß eine Strömungsverbindung zwischen dem zweiten Leitungsstück 30 und dem Behälter 14 durch das rohrförmige Anschlußstück 18 hindurch geschaffen wird.

Schließlich ist ein drittes Leitungsstück 36 vorgesehen, das vom Verknüpfungspunkt 28 abgeht und in das ein drittes Ventil 38 eingeschaltet ist. Dieses dritte Leitungsstück 36 mündet in eine Einrichtung zur Herstellung einer Dialysierflüssigkeit 40.

Diese Einrichtung 40 ist nur schematisch dargestellt, wobei die weiteren Konzentratzuführungen, sofern solche eingesetzt werden, nicht gezeigt sind. Lediglich dargestellt ist eine weitere Wasserleitung 42, die von der Wasserquelle 12 abgeht und direkt mit der Einrichtung 40 verbunden ist. Dort wird das über die Leitungen 30 und 36 zugeführte Flüssigkonzentrat in einem vorbestimmten Verhältnis mit Wasser vermischt. Anschließend wird die fertiggestellte Flüssigkeit über eine Dialysatleitung 44 dem Dialysegerät 46 zugeführt.

Des weiteren ist eine Einrichtung zur Eliminierung des Totvolumens 48 vorgesehen, die strichliert dargestellt ist.

Gemäß einer bevorzugten Ausführungsform weist diese Einrichtung zur Eliminierung des Totvolumens 48 eine Proportionierpumpe 50 auf, die über ein viertes Leitungsstück 52 mit dem Verknüpfungspunkt 28 verbunden ist. Die Proportionierpumpe 50 ist dabei vorteilhafterweise eine Kolbenpumpe mit definiertem Innenvolumen bzw. Fördervolumen.

Gemäß einer weiteren Ausführungsform kann die Proportionierpumpe 50, die zunächst als Einrichtung zur Eliminierung des Totvolumens dient, auch als reine Förderpumpe für Frischwasser eingesetzt werden.

Schließlich ist eine Steuereinheit 54 vorgesehen, die über Steuerleitungen 56,58,60,62,64 und 66 mit der Wasserquelle 12, dem ersten, zweiten bzw. dritten Ventil 26,32,38, der Einrichtung zur Eliminierung des Totvolumens 48 und der Einrichtung zur Herstellung der Dialyiserflüssigkeit 40 verbunden ist. Des weiteren ist im dritten Leitungsstück 36 eine Sensoreinheit 68 vorgesehen, die über eine Signalleitung 70 mit der Steuereinheit verbunden ist. Diese Sensoreinheit 56 kann die Konzentration des im Flüssigkonzentrat gelösten Salzes zumindest qualitativ, vorzugsweise jedoch aber auch quantitativ mittels Messung der aktuellen Elektrolytkonzentration und des Durchflusses durch das Leitungsstück 36 ermitteln.

Die in Fig. 1 gezeigte Ausführungsform einer Einrichtung zur Herstellung eines Flüssigkonzentrats 10 arbeitet folgendermaßen.

In der Füllphase wird aus der Wasserquelle 12 Frischwasser durch das erste Leitungsstück 24 bei aktiviertem ersten Ventil dem Verknüpfungspunkt 28 zugeführt. Vorteilhafterweise liefert die Wasserquelle 12 unter Druck, beispielsweise bis zu einem Druck von etwa 0,3 bar RO-Wasser, das bei aktiviertem zweiten Ventil 32 durch das zweite Leitungsstück 30 und das rohrförmige Anschlußstück 18 in den Beutel 14 durch die im Beutel vorgesehene Trockenkonzentratschicht hindurch gefördert wird. Die zugeführte Wassermenge wird letztlich durch das Innenvolumen des Beutels 14 bestimmt. Diese Menge kann entweder zeitgesteuert (durch die Öffnungszeiten der Ventile 26 und 32) eingestellt werden, wenn die Flußmenge des Wassers je Zeiteinheit bekannt ist. Andererseits reicht ein Druck von 0,2-0,3 bar nicht aus, um den Beutel 14 zum Platzen zu bringen, so daß auch eine reine Zeitsteuerung der Ventile 26 und 32 ausreichend erscheint.

Steht die Wasserquelle 12 nicht unter Druck, so muß das Wasser mittels einer nicht gezeigten, in der Wasserquelle 12 befindlichen Pumpe dem ersten Leitungsstück 24 zugeführt werden.

Andererseits ist es jedoch aber auch möglich, die volumetrische Pumpe 50 als Förderpumpe einzusetzen. In diesem Fall werden die beiden Ventile 26 und 32 gegenläufig getaktet, und zwar wird das Ventil 26 in der Füllphase der Pumpe 50 geöffnet, worauf sich beim Entleeren der Pumpe das zweite Ventil 32 öffnet, so daß das gesamte, vorher in die Pumpe 50 geförderte Wasser zum Beutel 14 transportiert wird.

Dabei ist das Innenvolumen der Pumpe 50 so bemessen, daß der Beutel 14 in maximal 20-30 Pumphüben gefüllt werden kann. Andererseits ist das Fördervolumen der Pumpe 50 mindestens doppelt so groß wie das nachstehend erläuterte Totvolumen der Leitungsanordnung 24,30,18,36.

Die Durchströmung des pulverförmigen Konzentrats 16 vom rohrförmigen Anschlußstück 18 her erfolgt so, daß sich nach dem Durchströmen des Pulvers im restlichen Innenraum 15 des Behälters 14 flüssiges Konzentrat im gesättigten Zustand befindet. Dieser Sättigungsvorgang wird im übrigen noch dadurch unterstützt werden, daß beim Ablassen des Flüssigkonzentrats, nachdem das erste Ventil 26 geschlossen und anstelle dessen das zweite Ventil 32 und das dritte Ventil 38 geöffnet worden sind, erneut die Festkonzentratschicht 16 durchströmt wird. Dabei strömt dann in der Entleerphase das Flüssigkonzentrat durch das Anschlußstück 18, das zweite Leitungsstück 30 und das dritte Leitungsstück 36 zur Einrichtung zur Herstellung der Dialysierflüssigkeit 40, das üblicherweise eine Ansaugeinrichtung in Form einer nicht gezeigten volumentrischen Pumpe besitzt. In diesem Stadium der Entleerung des Behälters 14 erhält dabei die Einrichtung 40 von der Steuereinrichtung 54 über die Steuerleitung 66 ein entsprechendes Aktivierungssignal.

Um die Sättigung der Konzentratlösung durchgehend aufrechtzuerhalten, muß das Totvolumen innerhalb der Leitungsanordnung beseitigt werden. Dieses Totvolumen setzt sich aus den Leitungsbestandteilen zusammen, durch die sowohl Wasser als auch Flüssigkonzentrat gefördert werden, also überwiegend das zweite Leitungsstück 30 sowie die Bereiche, die sich um den Verknüpfungspunkt 28 mit den Leitungsabschnitten 24,36 und ggf. 52 befinden.

Gemäß einer ersten Ausführungsform ist die Einrichtung zur Eliminierung des Totvolumens 58 als Pumpe 50 ausgebildet, die das Totvolumen über das vierte Leitungsstück 52 bei geöffnetem Ventil 32 absaugt. Am Ende dieser Absaugphase steht am Verknüpfungspunkt 28 gesättigtes Flüssigkonzentrat zur weiteren Verarbeitung zur Verfügung. Dabei kann die Pumpe 50, sofern sie ein ausreichendes Innenvolumen aufweist, das Totvolumen innerhalb der Pumpe 50 speichern. Wird der Beutel 14 wieder in die Füllphase geschaltet, so erhält die Pumpe 50 von dem Steuergerät 54 den Befehl, sich wieder zu entleeren, d.h. das gespeicherte Volumen Flüssigkeit in den Beutel 14 abzugeben.

Die Pumpe 50 dient bei dieser Ausführungsform als Mittel zur Entfernung des Totvolumens aus dem gemeinsamen Leitungsabschnitt 30, wobei die Speicherung dieses Flüssigkeitsvolumens innerhalb der Pumpe 50 erfolgt.

Die Pumpe 50 kann natürlich auch unmittelbar in einen Abfluß fördern, sofern sie über einen Ausgang verfügt. Des gleichen kann anstelle der Pumpe 50 ein strichliert dargestelltes Ventil 53 im Leitungsabschnitt 52 vorgesehen sein, sofern das Flüssigkonzentrat unter Druck aus dem Beutel 14, beispielsweise durch Schwerkraft bei dem in Fig. 1 gezeigten Beutel, bei geöffnetem Ventil 32 abgefördert werden. In einem solchen Fall kann bei geöffnetem Ventil 32 und bei geöffnetem Ablaßventil 53 über einen vorbestimmten Zeitraum hinweg die in der Totvolumenzone befindliche Flüssigkeit in den Abfluß befördert werden.

Wird die Pumpe 50 - wie bereits vorstehend erwähnt - auch zur Füllung des Beutels 14 eingesetzt, so erfolgt der Ausstoß des Totvolumens beim ersten Pumphub. Anschließend wird Frischwasser bei geöffnetem Ventil 26 angesaugt.

Gemäß einer weiteren Ausführungsform der Einrichtung zur Eliminierung des Totvolumens 48 dient die Pumpe 50 als Einrichtung zur vollständigen Durchmischung des Inhalts des Beutels 14 unter Einschluß des im Totvolumen befindlichen Wassers. Hierzu wird die Pumpe 50 mehrfach bei geschlossenem Ventil 26 und geöffnetem Ventil 32 gefüllt und entleert. Dies hat zur Folge, daß sowohl das Totvolumen als auch bereits fertiges Flüssigkonzentrat in die Pumpe 50 gefördert und dort durchmischt werden. Anschließend wird dieses Gemisch in den Beutel 14 ausgestoßen, so daß dort eine weitere Aufkonzentrierung erfolgt. Nachdem mehrere derartige Mischoperationen durchgeführt worden sind, befindet sich im gesamten Leitungssystem 18,30,52,36 sowie innerhalb der Pumpe 50 gesättigtes Flüssigkonzentrat.

Hierauf kann dann die Konzentratbildung abgeschlossen werden, wobei die Pumpe 50 desaktiviert und das Ventil 38 geöffnet wird.

Gemäß einer weiteren Ausführungsform kann bei geöffnetem Ventil 38 die Einrichtung 40 zur Herstellung der Dialysierflüssigkeit von der Steuereinrichtung 54 den Befehl erhalten, einen vorbestimmten Anteil der über die Leitung 30 und 36 zu befördernden Flüssigkeit als Wasser und nicht als Flüssigkonzentrat der in der Einheit 40 befindlichen Mischkammer zuzuführen. Hierzu wird vorteilhafterweise zuvor der Leitungsabschnitt 36 durch Betätigen des Ventils 26 und 38 von restlichem Flüssigkonzentrat befreit, so daß sich im gesamten Zuführungsabschnitt für das Flüssigkonzentrat zunächst Wasser befindet. Da üblicherweise die Zuführungsraten mittels der in der Einrichtung 40 vorgesehenen Pumpe und das Totvolumen bekannt sind, kann das Eintreffen der Konzentratfront in der Einheit 40 vorausbestimmt werden.

Andererseits kann jedoch aber auch mittels der Sensoreinheit 68 diese Front ermittelt werden, so daß durch ein entsprechendes Signal über die Leitungen 70 und 66 die Einheit 40 entsprechend aktiviert werden kann. Handelt es sich bei der Einheit 40 um einen Proportioniermischer, so läßt sich bei bekanntem Totvolumen bzw. bekannter Zuführrate aus dem Behälter 14 der Proportionalmischer so einstellen, daß die gewünschte Dialysierflüssigkeitszusammensetzung erzielt wird. In diesem Fall wird das Totvolumen zunächst als Wasser angesehen, so daß entsprechend weniger Wasser über die Wasserleitung (bei einer 1 : 34 bzw. 1 : 16 Mischung von Konzentrat mit Wasser} zugeführt wird. Am Mischpunkt erfolgt dann rechnerisch die gewünschte Zuführung von Konzentrat zur vorbestimmten Zusammensetzung der Dialysierflüssigkeit.

Das Ende der Entleerungsphase des Beutels 14 kann auf unterschiedliche Weise festgestellt werden. Wenn ein volumetrisches Mischsystem in der Einrichtung 40 zur Herstellung der Dialysierflüssigkeit eingesetzt wird, so läßt sich bei bekanntem Füllvolumen des Beutels 14 die Entleerung durch die von der Einrichtung 40 entnommenen Volumina genau feststellen. Infolgedessen wird nach Erreichen eines vorbestimmten Volumens ein entsprechendes Signal über die Leitung 66 an die Steuereinheit 54 von der Einrichtung 40 abgegeben.

Gleiches läßt sich auch bei proportionalen Mischsystemen in der Einrichtung 40 feststellen, deren Pumprate bei der Förderung von Flüssigkonzentrat zu einem in der Einheit 40 vorgesehen Mischpunkt bekannt ist. Auch hier läßt sich durch eine reine Zeitsteuerung die entnommene Flüssigkonzentratmenge feststellen, so daß die Steuereinheit 54 ebenfalls von der Einheit 40 nach Ablauf dieser Zeit aktiviert wird.

Eine weitere Ausführungsform zur Bestimmung des Endes der Entleerungsphase besteht darin, daß die Steuereinheit 54 ein Zeitglied aufweist, das nach Ablauf einer vorbestimmten Zeitdauer die Entleerungsphase beendet und die Füllphase wieder einleitet. Dies ist dann zweckmäßig, wenn die pro Zeiteinheit zugeführten Flüssigkonzentratmengen und die in den Beutel 14 in der Füllphase geförderten Wassermengen bekannt sind.

Andererseits kann jedoch aber auch die Füllgrad unmittelbar über einen Sensor 85 bestimmt werden, der über eine Signalleitung 87 mit der Steuereinheit 54 verbunden ist. Dieser Sensor 85 kann ein am Beutel 14 angebrachter Längensensor sein, mit dem der Ausdehnungsgrad des Beutels sowohl beim Befüllen als auch beim Entleeren festgestellt werden kann. Insofern kann ein solcher Längensensor sowohl den Füllvorgang als auch den Entleerungsvorgang in vorbestimmter Weise ermitteln.

Andererseits kann jedoch aber auch dieser Sensor 85 gravimetrisch das Gewicht des Behälters 14 bestimmen, so daß vorteilhafterweise vorbestimmte Flüssigkeitsmengen durch Gewichtsmessung bei der Zuführung sowie der Abführung ermittelt werden können.

Am Ende der Entleerungsphase wird dabei das dritte Ventil 38 geschlossen, wobei das zweite Ventil 32 geöffnet bleibt. Zugleich wird die Wasserquelle 12, sofern sie nicht aktiviert ist, und das erste Ventil 26 aktiviert, so daß wiederum Frischwasser zum Beutel 14 zufließen und diesen in der Füllphase füllen kann.

Das Ende der Füllphase kann, wie vorstehend erwähnt, durch den Sensor 85 ermittelt werden, so daß die Steuereinheit 54 zumindest das erste Ventil 26 schließt und den Entleerungsvorgang einleitet. Andererseits ist jedoch aber auch eine reine Zeitsteuerung möglich, wenn sichergestellt ist, daß das von der Wasserquelle 12 zugeführte Wasser den Beutel 14 nach dem vollständigen Füllen nicht zum Platzen bringt. Dies ist dann gewährleistet, wenn der Druck, mit dem das Wasser von der Wasserquelle 12 zugeführt wird, unterhalb des Berstdrucks des Beutels 14 liegt, üblicherweise unterhalb 0,2-0,3 bar.

Schließlich ist auch eine reine Zeitsteuerung des Füllvorgangs möglich, sofern die pro Zeiteinheit zugeführten Wassermengen und das Füllvolumen des Beutels 14 bekannt sind. Bei einer solchen Steuerung wird das erste Ventil 26 über eine vorbestimmte Zeit offengehalten und anschließend geschlossen. An die Füllphase schließt sich dann die Phase zur Eliminierung des Totvolumens an, nach deren Abschluß die Entleerungsphase beginnt.

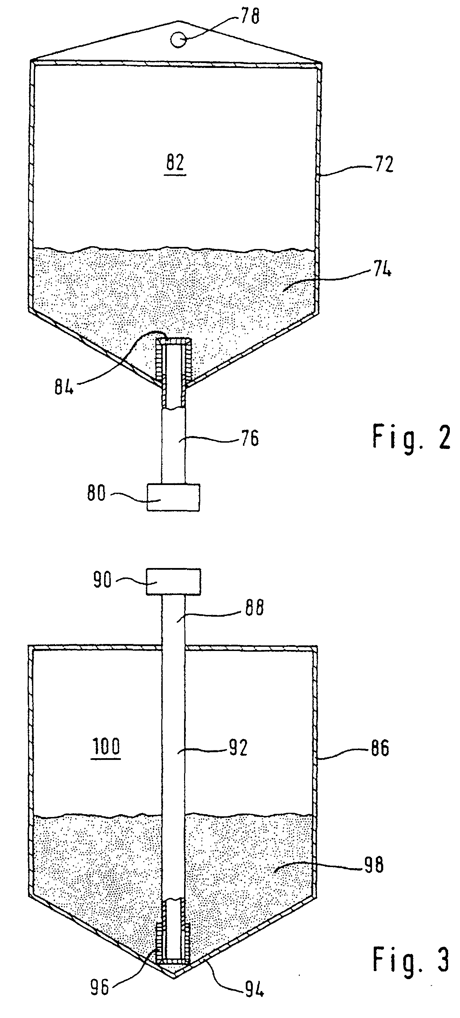

In Fig. 2 ist eine erste Ausführungsform eines erfindungsgemäßen Behälters 14, wie er bereits in Fig. 1 gezeigt ist, in Form eines Beutels 72 gezeigt, der mit einem trockenen Elektrolytkonzentrat, vorzugsweise Natriumbicarbonat in einer solchen Menge gefüllt ist, daß die Behandlung eines Patienten während einer Sitzung gewährleistet ist. Üblicherweise liegt diese Menge zwischen 500 und 800 g Bicarbonat, das vorteilhafterweise eine mittlere Kornung von 0,5 mm und darüber aufweist. Diese Konzentratschicht ist mit 74 bezeichnet.

Bei dem in Fig. 2 gezeigten Beutel 72 handelt es sich um ein Hängesystem, bei dem der rohrförmige Anschußstutzen 76 in der Gebrauchslage unten angeordnet ist. Gegenüber dem Anschlußstutzen 76 befindet sich eine Aufhängeöse 78 im Rand des Beutels 72.

Des weiteren ist der Anschlußstutzen 76 mit einem Konnektorstück 80 versehen, wie es bereits vorstehend erwähnt worden ist. Durch den Anschlußstutzen 76 hindurch besteht eine Strömungsverbindung mit dem Innenraum 82 des Beutels, der üblicherweise zu 1/3-1/4 seines Volumens mit dem Konzentrat gefüllt ist. Der Rest des Innenraums 82 kann mittels Wasser bzw. gesättigter Konzentratlösung aufgefüllt sein.

Das im Innenraum 82 liegende Ende des Anschlußstutzens 76 ist mit einer halbdurchlässigen Membranschicht oder Filterschicht 84 verschlossen, die die Eigenschaft hat, einerseits zuströmendes Wasser und abströmende Konzentratlösung durchzulassen, andererseits Konzentratpulver oder -granulat am Abströmen durch den Anschlußstutzen 76 hindurch zu hindern. Ein Filter hält beispielsweise sämtliche Partikel zurück, die eine Korngröße von mehr als 0,3 µm aufweisen. Kleinere Partikel können dagegen im Konzentratstrom mitgerissen werden und lösen sich spätestens in der Einrichtung 40 auf. Ihre Konzentration ist derart gering, daß sie keine Nennenswerte Änderung der Zusammensetzung der Dialysierflüssigkeit nach sich ziehen.

In Fig. 3 ist eine weitgehend der Ausführungsform von Fig. 2 ähnliche Form gezeigt, wobei es sich hier um einen Beutel 86 handelt, bei dem der Anschlußstutzen 88 in der Gebrauchslage oben ist. Dabei kann das Konnektorstück 90 nicht nur zum Konnektieren, sondern auch zum Befestigen des Beutels 86 an dem nicht gezeigten Dialysegerät dienen.

Der Beutel 86 weist dabei ein Tauchrohr 92 auf, das sich im Anschluß an den Anschlußstutzen 88 bis zum Bodenbereich 94 erstreckt, der gemäß der in Fig. 3 gezeigten Ausführungsform vorteilhafterweise konisch nach unten spitz zulaufend verläuft.

Das Ende des Tauchrohrs 92 ist mit der gleichen Membran- oder Filterschicht 96 verschlossen, wie er mit 84 bei der Ausführungsform von Fig. 2 gezeigt ist.

Diese Filterschicht 96 befindet sich in der pulverförmigen Konzentratschicht 98, die der Konzentratschicht 74 gleicht.

Die Ausführungsformen von Fig. 2 und 3 haben den Vorteil, daß die in die Beutel 72 und 86 beförderte Flüssigkeit zweimal die Konzentratschicht 74 und 98 durchqueren muß. So fließt das Wasser zunächst beim Zufördern durch die Konzentratschicht hindurch und sättigt sich weitgehend bzw. vollständig mit Natriumbicarbonat. Wird anschließend Lösung aus dem Innenraum 82 bzw. 100 des Beutels 86 abgepumpt, so muß die aus dem Innenraum nachstehende Lösung nochmals durch die Konzentratschicht hindurch und löst, sofern sie nicht bereits gesättigt ist, pulverförmiges Konzentratsalz bis zu ihrer Sättigung auf.

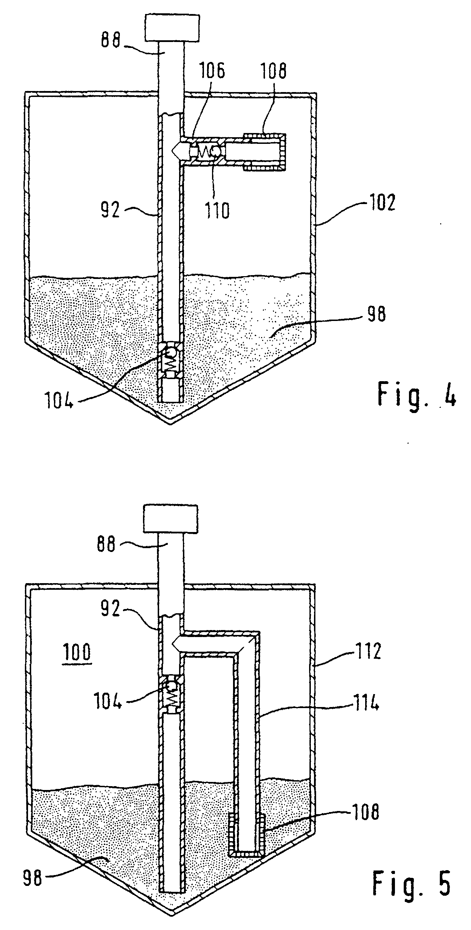

In Fig. 4 ist eine dritte Ausführungsform eines Beutels 102 gezeigt, der im wesentlichen der Ausführungsform gemäß Fig. 3 entspricht, so daß auf die Ausführungsform gemäß Fig. 3 mit ihren Bezugszeichen Bezug genommen wird. Bei der in Fig. 4 gezeigten Ausführungsform wird jedoch nur der Zufluß von Wasser durch das Tauchrohr 92 bis zu seinem Ende zugelassen, denn ein Einwegventil 104 sperrt den Rückfluß in Richtung Anschlußstück 88. Infolgedessen ist benachbart zum Anschlußstück 88 im Innenraum 100 ein Abflußstutzen bzw. eine Öffnung 106 am Tauchrohr 92 vorgesehen, durch das Flüssigkonzentrat abgezogen werden kann. Gemäß einer ersten Ausführungsform ist das Ende des Abflußstutzens 106 mit einer Membran bzw. Filterschicht 108 verschlossen, die den vorstehend genannten Filterschichten 84 und 96 entspricht. Gemäß einer anderen Ausführungsform ist in dem Abflußstutzen ein zweites Rückschlagventil 110 vorgesehen, das nur den Abfluß in Richtung Anschlußstutzen 88 zuläßt, ansonsten aber den Zufluß von Wasser sperrt.

Eine weitere Ausführungsform eines Beutels 112 ist in Fig. 5 dargestellt, die weitgehend der Ausführungsform gemäß Fig. 4 bzw. Fig. 3 entspricht, so daß auch hier wieder die gleichen Bezugszeichen für gleiche Teile eingesetzt werden.

Anstelle des Abflußstutzens 106 ist ein zweites Tauchrohr 114 vorgesehen, das mit der Membran 108 verschlossen ist. Dieses Tauchrohr durchsetzt die Konzentratschicht 98 und ist an ihrem Ende wiederum mit der Filterschicht 108 verschlossen. Andererseits zweigt sie benachbart vom Anschlußstutzen 88 innerhalb des Beutels 112 vom Rückschlagventil 104 im ersten Tauchrohr 92 ab.

Dabei läßt das erste Rückschlagventil 104 den Zufluß von Wasser durch den Innenraum 100 und die Konzentratschicht 98 zu, sperrt jedoch den Rückfluß. Dieser erfolgt dann durch die Filterschicht 108, das zweite Tauchrohr 114, den oberen Teil des ersten Tauchrohrs 92 und den Anschlußstutzen 88.

Die Beutel 72,86,102 und 112 sind jeweils eine selbständige Handelseinheit und können bei Bedarf an die Einrichtung 10 angeschlossen werden. Dabei ist unter Beutel nicht nur der übliche Kunststoffbeutel, der vorzugsweise aus einem klaren durchsichtigen Material besteht, sondern auch ein Behälter mit festen Wänden zu verstehen, der dann über ein Belüftungsmittel, üblicherweise eine hydrophobe Membran, die Luft, nicht jedoch Wasser durchläßt, verfügt. Ein solcher Behälter kann ebenfalls aus einem klaren Kunststoffmaterial oder aber aus Glas bestehen. Solche Gefäße mit festen Wänden können dann zweckmäßig sein, wenn Konzentrate aufgelöst werden, bei denen kein Gas, wie bei Bicarbonat durch Disproportionierung von Bicarbonat in Carbonat und CO₂, entsteht. Insofern ist für die Auflösung von Bicarbonat ein Behälter mit festen Wänden nicht bevorzugt.

## Patentansprüche

1. Vorrichtung zur Herstellung eines flüssigen Hämodialysekonzentrats mit einer Wasserquelle (12), einem Behälter (14) mit einem einzigen Anschlußstück (18,76,88), der ausreichend pulverförmiges Konzentratsalz (16) fiir eine Dialysebehandlung aufweist, einer Leitungsanordnung (24,30,36), die einerseits die Wasserquelle (12) und andererseits das Anschlußstück (18) sowie eine Einrichtung (40) zur Herstellung von Dialysierflüssigkeit verbindet, einer Absperranordnung (26,32,38), die jeweils in vorbestimmter Weise die Leitungsanordnung (24,30,36) freigibt bzw. sperrt, sowie mit einer Steuereinheit (54) zur Aktivierung der Wasserquelle (12), der Absperranordnung (26,32,38) und der Einrichtung (40) zur Herstellung von Dialysierflüssigkeit, **dadurch gekennzeichnet, daß** eine Einrichtung (48) zur Kompensierung eines Totvolumens, das im wesentlichen durch den gemeinsam von Wasser und Flüssigkonzentrat durchflossenen Leitungsabschnitt (18,30) gebildet ist, vorgesehen ist, die vor Beginn der Entleerungsphase durch die Steuereinheit (54) aktiviert wird.

2. Vorrichtung nach Anspruch 1, **dadurch gekennzeichnet, daß** die Leitungsanordnung ein erstes Leitungsstück (24), das von der Wasserquelle (12) zu dem Verknüpfungspunkt (28) führt, ein zweites Leitungsstück (30), das von dem Anschlußstutzen (18) zu dem Verknüpfungspunkt (28) führt, ein drittes Leitungsstück (36), das von der Einrichtung (40) zur Herstellung von Dialysierflüssigkeit zum Verknüpfungspunkt (28) führt, und ein von dem Verknüpfungspunkt (28) abgehendes viertes Leitungsstück (52) aufweist, das mit der Einrichtung (48) zur Eliminierung des Totvolumens schaltmäßig verbunden ist, wobei die Absperranordnung ein erstes Ventil (26) in dem ersten Leitungsstück (24), ein zweites Ventil (32) in dem zweiten Leitungsstück (30) und ein drittes Ventil (38) in dem dritten Leitungsstück (36) aufweist.

3. Vorrichtung nach Anspruch 2, **dadurch gekennzeichnet, daß** die Einrichtung (48) zur Eliminierung des Totvolumens eine Proportionierpumpe (50) ist, die von der Steuereinheit (54) aktivierbar ist.

4. Vorrichtung nach Anspruch 3, **dadurch gekennzeichnet, daß** die Proportionierpumpe (50) von der Steuereinheit (54) bei geöffnetem zweiten Ventil (32) und geschlossenem ersten und dritten Ventil (26, 38) in der Eliminationsphase so betrieben ist, daß sie zumindest das gesamte Totvolumen absaugt und bis zum nächsten Füllschritt speichert.

5. Vorrichtung nach Anspruch 3, **dadurch gekennzeichnet, daß** die Proportionierpumpe (50) in der Eliminationsphase bei geöffnetem zweiten Ventil (32) und geschlossenem ersten und dritten Ventil (26, 38) von der Steuereinheit (54) wechselweise in den Ansaug- und Entleerungsbetrieb geschaltet ist.

6. Vorrichtung nach Anspruch 2, **dadurch gekennzeichnet, daß** die Einrichtung (48) zur Eliminierung des Totvolumens ein viertes Ventil (53) ist, das in der Eliminationsphase zusammen mit dem zweiten Ventil von der Steuereinheit (54) über eine solche Zeitdauer geöffnet ist, daß das Totvolumen durch das vierte Leitungsstück (52) abgeführt wird.

7. Vorrichtung nach Anspruch 1, **dadurch gekennzeichnet, daß** die Einrichtung (40) zur Herstellung der Dialysierflüssigkeit ein Proportioniermischer mit einer Mischkammer zum Mischen von Wasser und Flüssigkonzentrat in einem vorgegebenen Mischungsverhältnis ist, wobei die Einrichtung (48) zur Eliminierung des Totvolumens die Einrichtung (40) zur Erzeugung der Dialysierflüssigkeit so ansteuert, daß die Einrichtung (40) zur Herstellung der Dialysierflüssigkeit die im Totvolumen befindliche Flüssigkeitsmenge der Mischkammer nicht als Flüssigkonzentrat, sondern als Wasser zuführt.

8. Vorrichtung nach Anspruch 7, **dadurch gekennzeichnet, daß** die Einrichtung (48, 54) zur Eliminierung des Totvolumens eine Sensoreinheit (68) aufweist, die fortlaufend in der Eliminationsphase die Zusammensetzung der das dritte Leitungsstück (36) passierenden Flüssigkeit bestimmt und das Erreichen eines vorbestimmten Meßwerts hinsichtlich Zusammensetzung und/oder Menge der Flüssigkeit der Steuereinheit (54) signalisiert, die dann die Einheit (40) zur Herstellung der Dialysierflüssigkeit in die Phase der Förderung von Flüssigkonzentrat umschaltet.

9. Vorrichtung nach einem der Ansprüche 2 bis 8, **dadurch gekennzeichnet, daß** die Steuereinheit (54) die Einrichtung (40) zur Herstellung der Dialysierflüssigkeit am Ende der Entleerungsphase desaktiviert, das dritte Ventil (38) schließt und die Wasserquelle (12) sowie das erste und zweite Ventil (26,32) in vorbestimmter Weise zur Einleitung der Füllphase aktiviert.

## Claims

1. Device for the manufacture of a liquid haemodialysis concentrate with a supply of water (12), a container (14) with a single connection tube (18, 76, 88) which possesses a sufficient supply of powdered concentrate salt (16) for a dialysis treatment, a tubing arrangement (24, 30, 36) one side of which is connected to the water supply (12) and the other to the connection tube (18) together with a unit (40) for the production of the dialysing liquid, a flow-control device (26, 32, 38) which in a pre-determined manner permits or blocks the flow in the tubing arrangement (24, 30, 36) together with a control unit (54) to activate the supply of water (12), the flow-control device (26, 32, 38) and the unit (40) for the manufacture of the dialysing liquid, **characterised in that** a unit (48) is provided for the compensation of a dead volume which, basically, is formed by the section of tubing (18, 30) through which both water and the liquid concentrate flow, which device is activated by the control unit (54) before the commencement of the emptying phase.

2. Device in accordance with claim 1, **characterised in that** the tubing arrangement exhibits a first tube element (24) which leads from the source of water (12) to the connecting point (28), a second tube element (30) which leads from the connection tube (18) to the connecting point (28), a third tube element (36) which leads from the unit (40) for producing the dialysing liquid to the connecting point (28) and a fourth tube element (52) leaving the connecting point (28) which is connected in a switchable manner to the unit (48) for the elimination of the dead volume, such that the flow control device exhibits a first valve (26) in the first tube element, a second valve (32) in the second tube element (30) and a third valve (38) in the third tube element (36).

3. Device in accordance with claim 2 **characterised in that** the unit (48) for eliminating the dead volume is a proportioning pump (50) which can be activated by the control unit (54).

4. Device in accordance with claim 3, **characterised in that** during the elimination phase when the second valve (32) is open and the first and third valves (26 and 38) are closed, the proportioning pump (50) is so driven by the control unit (54) that it sucks out at least the total dead volume and stores this until the subsequent filling step.

5. Device in accordance with claim 3, **characterised in that** during the elimination phase, when the second valve (32) is open and the first and third valves (26 and 38) are closed, the proportioning pump (50) is alternately switched by the control unit (54) between the sucking operation and the emptying operation.

6. Device in accordance with claim 2, **characterised in that** the unit (48) for eliminating the dead volume is a fourth valve (53) which during the elimination phase is opened together with the second valve by the control unit (54) over such a period of time that the dead volume is conducted away through the fourth tube element (52).

7. Device in accordance with claim 1, **characterised in that** the unit (40) for the production of the dialysing liquid is a proportioning mixer with a mixing chamber for mixing water and liquid concentrate in a predetermined ratio, whereby the unit (48) for the elimination of the dead volume so triggers the unit (40) for the production of the dialysing liquid that the unit (40) for the production of the dialysing liquid supplies the quantity of liquid of the mixing chamber found in the dead volume as water rather than as liquid concentrate.

8. Device in accordance with claim 7, **characterised in that** the unit (48, 54) for the elimination of the dead volume exhibits a sensor unit (68) which during the elimination phase continuously determines the composition of the liquid passing through the third tube element (36) and passes a signal to the control unit (54) when a predetermined measured value relating to the composition and / or quantity of the liquid is attained whereupon the control unit (54) then switches the unit (40) for the production of the dialysing liquid into the phase of supplying the liquid concentrate.

9. Device in accordance with one of the claims 2 to 8, **characterised in that** the control unit (54) deactivates the unit (40) for the manufacture of the dialysing liquid at the end of the emptying phase, closes the third valve (38) and activates the supply of water (12) together with the first and second valves (26, 32) in a predetermined manner to introduce the filling phase.

## Revendications

1. Dispositif pour préparer un concentrat d'hémodialyse ayant une source d'eau (12), un contenant (14) avec une unique partie raccord (18, 76, 88), présentant suffisamment de sel de concentrat pulvérulent (16) pour un traitement de dialyse, un système de tuyaux (24, 30, 36) et un appareil (40) destiné à préparer le liquide de dialyse, un système d'arrêt (26, 32, 38) qui libère ou bloque le système de tuyaux (24, 30, 36) respectivement de manière prédéterminée, et ayant une unité de commande (54) pour activer la source d'eau (12), le système d'arrêt (26, 32, 38) et l'appareil (40) destiné à préparer le liquide de dialyse, **caractérisé en ce que** l'on prévoit un dispositif (48) pour compenser un volume mort qui est formé essentiellement par la section de tuyau commune (18, 30) traversée par l'eau et le concentrat liquide, activée avant le début de la phase de vidange par l'unité de commande (54).

2. Dispositif selon la revendication 1, **caractérisé en ce que** le système de tuyaux présente une première partie de tuyau (24) qui conduit de la source d'eau (12) au point de jonction (28), une deuxième partie de tuyau (30) qui conduit du tuyau de rallonge (18) au point de jonction (28), une troisième partie de tuyau (36) qui conduit de l'appareil (40) destiné à préparer le liquide de dialyse au point de jonction (28) et une quatrième partie de tuyau (52) partant du point de jonction(28) qui est relié au dispositif (48) destiné à éliminer le volume mort par commutation, le système d'arrêt présentant un premier robinet (26) dans une première partie de tuyau (24), un deuxième robinet (32) dans une deuxième partie de tuyau (30) et un troisième robinet (38) dans une troisième partie de tuyau (36).

3. Dispositif selon la revendication 2, **caractérisé en ce que** le dispositif (48) destiné à éliminer le volume mort est une pompe proportionnelle (50) pouvant être activée par l'unité de commande (54).

4. Dispositif selon la revendication 3, **caractérisé en ce que** la pompe proportionnelle (50) est actionnée par l'unité de commande (54) dans la phase d'élimination quand le deuxième robinet (32) est ouvert et les premier et troisième robinets (26, 38) sont fermés de telle manière qu'elle aspire au moins le volume mort total et le stocke jusqu'à la prochaine étape de remplissage.

5. Dispositif selon la revendication 3, **caractérisé en ce que** la pompe proportionnelle (50) est commutée par l'unité de commande (54) tour à tour en mode remplissage et vidange, quand le deuxième robinet (32) est ouvert et les premier et troisième robinets (26, 38) sont fermés.

6. Dispositif selon la revendication 2, **caractérisé en ce que** le dispositif (48) destiné à éliminer le volume mort est un quatrième robinet (53) qui est ouvert dans la phase d'élimination en compagnie du deuxième robinet (53) par l'unité de commande (54) pendant une durée telle que le volume mort est évacué par la quatrième partie de tuyau (52).

7. Dispositif selon la revendication 1, **caractérisé en ce que** l'appareil (40) destiné à préparer le liquide de dialyse est un mélangeur proportionnel doté d'une chambre de mélange pour mélanger l'eau et le concentrat liquide en un rapport de mélange prédéterminé, le dispositif (48) destiné à éliminer le volume mort commandant l'appareil (40) destiné à produire le liquide de dialyse de telle manière que l'appareil (40) destiné à préparer le liquide de dialyse n'amène pas la quantité de liquide de la chambre de mélange se trouvant dans le volume mort en tant que concentrat liquide mais en tant qu'eau.

8. Dispositif selon la revendication 7, **caractérisé en ce que** le dispositif (48, 54) destiné à éliminer le volume mort présente une unité de détection (68) qui détermine en continu dans la phase d'élimination la composition du liquide passant dans la troisième partie de tuyau (36) et signale l'atteinte d'une valeur de mesure prédéterminée au niveau de la composition et/ou de la quantité de liquide à l'unité de commande (54) qui ensuite fait passer l'unité (40) destinée à préparer le liquide de dialyse en phase de circulation du concentrat liquide.

9. Dispositif selon les revendications 2 à 8, **caractérisé en ce que** l'unité de commande (54) désactive l'appareil (40) destiné à préparer le liquide de dialyse à la fin de la phase de vidange, ferme le troisième robinet. (38) et active la source d'eau (12) ainsi que le premier et le deuxième robinet (26, 32) de manière prédéterminée pour enclencher la phase de remplissage.
